(19) Europäisches Patentamt
European Patent Office
Office européen des brevets

(11) **EP 2 992 848 B1**

(12) **EUROPÄISCHE PATENTSCHRIFT**

(45) Veröffentlichungstag und Bekanntmachung des Hinweises auf die Patenterteilung:
**28.12.2022 Patentblatt 2022/52**

(21) Anmeldenummer: **14183771.6**

(22) Anmeldetag: **05.09.2014**

(51) Internationale Patentklassifikation (IPC):
***A61B 18/12*** *(2006.01)*

(52) Gemeinsame Patentklassifikation (CPC):
**A61B 18/1233;** A61B 2018/00589;
A61B 2018/00642; A61B 2018/00672;
A61B 2018/00678; A61B 2018/00767;
A61B 2018/00875

(54) **EINRICHTUNG ZUR KONTAKTKOAGULATION VON BIOLOGISCHEM GEWEBE**

DEVICE FOR CONTACT COAGULATION OF BIOLOGICAL TISSUE

DISPOSITIF DE COAGULATION PAR CONTACT DE TISSU BIOLOGIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.03.2016 Patentblatt 2016/10**

(73) Patentinhaber: **Erbe Elektromedizin GmbH**
**72072 Tübingen (DE)**

(72) Erfinder:
• **Keller, Sandra**
**72379 Hechingen (DE)**
• **Kegreiss, Marc**
**72072 Tübingen (DE)**

(74) Vertreter: **Rüger Abel Patentanwälte PartGmbB**
**Patentanwälte**
**Webergasse 3**
**73728 Esslingen a. N. (DE)**

(56) Entgegenhaltungen:
RU-C2- 2 294 171        US-A1- 2002 165 531
US-A1- 2003 158 551

**Beschreibung**

Die Erfindung betrifft eine Einrichtung zur Kontaktkoagulation von biologischem Gewebe unter Einwirkung von elektrischem Strom.

Bei der Kontaktkoagulation von biologischem Gewebe laufen vorwiegend thermisch induzierte Vorgänge ab, die unter anderem zum Denaturieren des Gewebes führen, wobei vorhandene Hohlgefäße geschlossen werden sollen. Beim Anlegen einer HF-Spannung an ein biologisches Gewebe lässt sich zu Beginn des Vorgangs eine hohe elektrischer Impedanz des Gewebes beobachten. Die Stromleitung von elektrischen Ladungsträgern findet vorwiegend in extrazellulärer Flüssigkeit statt, wodurch sich das Gewebe aufgrund der kinetischen Energie der bewegten elektrischen Ladungsträger zu erwärmen beginnt. Mit zunehmender Erwärmung des Gewebes sinkt die Impedanz bis sie ein Minimum erreicht. Die Erhöhung der elektrischen Leitfähigkeit tritt in einem Temperaturbereich von 60°C bis 100°C des biologischen Gewebes aufgrund seiner temperaturinduzierten strukturellen Veränderungen, die mit der Denaturierung des Gewebes einhergehen, auf. Das Gewebe wird devitalisiert, es verklumpen Eiweißmoleküle, die Zellmembran wird zerstört, wodurch Gewebsflüssigkeit freigesetzt wird. In dieser "Phase I" nimmt die Gewebeimpedanz Z fortwährend ab. Nach einiger Zeit wird die Siedetemperatur der Gewebsflüssigkeit erreicht, womit der Gewebewiderstand wieder ansteigt, was als "Phase II" bezeichnet wird. Üblicherweise erreicht die Gewebeimpedanz Z in der Phase II Werte, die deutlich über dem Impedanzminimum des Gewebes und oftmals der Anfangsimpedanz aus Phase I liegen.

Aus der EP 2 520 240 A1 sind ein Verfahren und eine Einrichtung zur Gewebefusion und auch zur Koagulation bekannt, bei der durch Festlegung eines negativen Innenwiderstands einer speisenden HF-Quelle eine konstant gleichbleibende Behandlungszeit erreicht werden soll.

Aus der EP 1 862 137 A1 ist eine Vorrichtung und ein Verfahren zur Koagulation von Gewebe bekannt, bei dem die Gewebeimpedanz Z abgefragt und überwacht wird. Durch fortwährendes Nachstellen der an das Gewebe gelieferten elektrischen Energie wird erreicht, dass die Impedanz des Gewebes einer gewünschten vorgegebenen Kurve folgt. Dies betrifft insbesondere die Phase II.

Aus der US 2003/158551 A1 ist ebenfalls eine Koagulationseinrichtung mit Überwachung der Gewebeimpedanz bekannt. Die an das Gewebe angelegt Spannung wird graduell erhöht, während die Gewebeimpedanz abfällt und ein Minimum durchläuft. Wenn die Gewebeimpedanz danach einen bestimmten Wert erreicht, wir die angelegte Spannung gepulst.

Mit steigender Spannung während des Abfalls der Gewebeimpedanz und dem Durchlauf eines Impedanzminimums arbeitet auch die RU 2 294 171 C2.

Weiter ist aus der DE 36 22 337 A1 ein Hochfrequenzgenerator mit automatischer Leistungsregelung für die Hochfrequenzkoagulation bekannt, der eine Lichtbogenanzeigevorrichtung zur Feststellung eines zwischen der Koagulationssonde und dem Gewebe bestehenden Lichtbogens aufweist. Um den Lichtbogen sicher zu zünden, wird zunächst mit maximaler Ausgangsleistung gearbeitet. Nach dem Zünden des Lichtbogens wird zunächst weiterhin für eine bestimmte Zeitdauer die maximale Leistung abgegeben. Die Ausgangsleistung wird danach für eine vorbestimmte zweite Zeitdauer auf Null reduziert. Solange der Generator aktiviert ist, werden diese Zyklen stets wiederholt.

Mit dem in DE 36 22 337 A1 beschriebenen Verfahren wird ein Koagulationsmodus erreicht, bei dem die Koagulation zunächst mit Kontaktkoagulation beginnt, wobei nach Erreichen der Siedetemperatur der Gewebsflüssigkeit ein Lichtbogen den sich bildenden Dampf durchschlägt, wobei die Stromdichte an dem Durchschlagort bei dem Lichtbogen stark erhöht ist, wodurch ein ausgeprägter örtlicher Koagulationseffekt auftritt und das Gewebe hochohmig wird. Der Lichtbogen zündet und springt zu verschiedenen Stellen, bis das gesamte Gewebe in der Nachbarschaft der Koagulationssonde hochohmig geworden also koaguliert ist. Das zeitweilige Abschalten des Lichtbogens durch das Setzen der Ausgangsleitung auf Null verhindert das übermäßige Verbrennen, d.h. eine zu starke Karbonisierung des Gewebes.

Bei einer schnellen Koagulation, die auf einer Funkenbildung beruht oder diese zulässt, kann es zum Ankleben von Gewebe am Instrument und damit einhergehend zu einer zum Teil nicht unerheblichen Verschmutzung des Instruments und auch des Behandlungspersonals kommen. Außerdem kann die auftretende Karbonisierung den Wundheilungsprozess erschweren.

Wird eine schnelle Koagulation ohne Lichtbogen durch reine Kontaktkoagulation in der Phase I mit erhöhter HF-Leistungsabgabe eines HF-Generators bewirkt, kann ein akustisch hörbares und visuell wahrnehmbares Zerreißen von behandeltem Gewebe auftreten. Dies wird durch lokales Zerreißen von Gewebe aufgrund von siedender Gewebeflüssigkeit und damit einhergehender Gewebedruckzunahme hervorgerufen. Durch das Zerreißen des Gewebes können zuvor gestillte Blutungen erneut zu bluten beginnen. Außerdem kann behandeltes pathogenes Gewebe durch das Zerreißen in gesunde Gewebeareale streuen oder auch von Behandlungspersonal aufgenommen werden.

Es ist Aufgabe der Erfindung, ein Konzept anzugeben, mit dem sich eine schnelle Gewebekoagulation bei schonender Behandlung erreichen lässt.

Diese Aufgabe wird mit der Einrichtung nach Anspruch 1 gelöst. Weitere bevorzugte Ausführungsformen sind in den abhängigen Ansprüchen beschrieben.

Es hat sich gezeigt, dass das Zerreißen von bereits teilweise oder ganz koaguliertem Gewebe mit dem Durchschreiten des Miniums der Gewebeimpedanz $\underline{Z}$ einhergeht. Im Impedanzminimum hat das Gewebe seinen leitfähigsten Zustand erreicht. Dies tritt bereits vor Erreichen des Siedepunkts der Gewebsflüssigkeit auf, d.h. bevor eine Temperatur von 100°C erreicht ist. Danach kommt es bei angelegter HF-Spannung zum Verdampfen der Gewebsflüssigkeit. Eine solche Verdampfung führt zu einem Druckanstieg im Gewebe und zum Zerreißen desselben und wird bei der Erfindung vermieden. Dadurch wird auch vermieden, dass kontaminiertes Gewebe verstreut, offene Wunden und Instrumente kontaminiert und OP-Personal verschmutzt oder infiziert wird. Außerdem wird ein Zerreißen von bereits koagulierten Blutgefäßen, Lymphgefäßen oder anderen Gefäßen vermieden. Durch das Fortsetzen der Koagulation mit verringerter Spannung kann die Koagulation ohne solche Effekte fortgesetzt werden, bis sich der gewünschte Gewebeeffekt ausbildet.

Der vorgeschlagene neue Kontaktkoagulationsmodus arbeitet solange mit erhöhtem Leistungseintrag, bis das Impedanzminimum erreicht wird. Hierbei wird in der Phase I bis zum Erreichen des Impedanzminimums die maximal mögliche HF-Energie durch Abgabe einer hohen HF-Spannung in das Gewebe eingetragen. Der Beginn der Koagulation erfolgt bei der Berührung des Instruments mit dem Gewebe mit maximaler Leistungsabgabe des Generators, d.h. mit einer HF-Spannung, die unabhängig vom Strom vorzugsweise deutlich über 200V liegt. Die Entstehung von Dampf im Gewebe und somit das Zerreißen von Gewebe wird jedoch verhindert, indem die Applikation erhöhter HF-Spannung abgebrochen wird, sobald ein Impedanzminimum detektiert wird. Es wird danach lediglich eine reduzierte HF-Spannung bereitgestellt.

Bei der Erfindung ist in der Generatorsteuerung ein Einstellwert für die zu applizierende Spannung hinterlegt. Die Generatorsteuerung ist dann darauf eingerichtet, zu Beginn eines Koagulationsprogramms den Generator zu veranlassen, die zu applizierende Spannung mit einem Wert bereitzustellen, der größer ist als der Einstellwert. In der Erfindung ist die zu applizierende Spannung zu Beginn eines Koagulationsvorgangs mindestens doppelt so groß wie der Einstellwert. Wenn der Anwender die gewohnte, zur Kontaktkoagulation geeignete Spannung (z.B. 200 V) einstellt, arbeitet er bei dem erfindungsgemäßen System zu Beginn eines Koagulationsvorgangs mit einer Spannung von mindestens 400 V. Zur Funkenbildung kommt es jedoch deswegen nicht, weil die Spannung reduziert wird, sobald ein Impedanzminimum durchlaufen ist und Dampfbildung droht. Durch die Vermeidung von Dampfbildung kann auch die Funkenbildung, das damit einhergehende Platzen von Dampfblasen im Gewebe und damit einhergehende Nachteile vermieden werden.

Bei der Erfindung wird nach Erkennung des ersten Minimums der Gewebeimpedanz der Generator veranlasst, die zu applizierende Spannung mit einem Wert bereitzustellen, der nicht größer als der Einstellwert ist. Der Einstellwert wird dabei auf einen Wert festgelegt, der eine fortgesetzte Koagulation ohne Funkenbildung ermöglicht. Vorzugsweise ist die Generatorsteuerung darauf eingerichtet, in Reaktion auf die Erkennung eines zweiten oder weiteren Minimums der Gewebeimpedanz $\underline{Z}$ den Generator zu veranlassen, die zu applizierende Spannung auf einen Wert zu reduzieren, der kleiner als der Einstellwert ist. Vorzugsweise ist dieser Wert um einen vorgegebenen Prozentsatz, beispielsweise 10%, kleiner als die zuvor gelieferte Spannung.

Weiter kann die Generatorsteuerung darauf eingerichtet sein, die HF-Applikation des Generators zu beenden, wenn die zu applizierende Spannung einen Wert erreicht hat, der einen festgelegten Bruchteil des eingestellten Werts nicht überschreitet bzw. unterschreitet. Dieser festgelegte Bruchteil kann beispielsweise 60% der eingestellten Spannung betragen. Mit einer solchen Einrichtung wird eine schnelle großvolumige Koagulation erreicht. Der Einstellwert der zu applizierenden Spannung kann in Stufen oder stufenlos variable vorgebbar sein. Vorzugsweise sind dazu entsprechende Einstellmittel an dem das Instrument speisenden Gerät vorgesehen. Die Überwachung der Gewebeimpedanz kann kontinuierlich oder in eng aufeinanderfolgenden Zeitpunkten quasi kontinuierlich erfolgen. Vorzugsweise sind die Zeitabstände zwischen einzelnen Messungen des Gewebewiderstands geringer als 0,2 ms. Vorzugsweise betragen die Zeitabstände etwa 100 ps. Damit kann sehr schnell auf den sich ändernden Gewebewiderstand reagiert werden.

Bei der Erfassung der aktuellen Gewebeimpedanz in kurzen Zeitabständen werden vorzugsweise die bereitgestellte applizierte Spannung und der durch das biologische Gewebe fließende Strom, sowie deren Phasenlage zueinander in Zeitfenstern erfasst, deren Länge mindestens so lang ist wie eine Schwingungsperiode, weiter vorzugsweise mindestens so lang wie mehrere Schwingungsperioden der HF-Spannung. Vorzugsweise werden dabei mindestens ein Kennwert der anliegenden Spannung und mindestens ein Kennwert des fließenden Stroms erfasst. Ein Kennwert der Spannung kann die Spitzen-Spitzen-Spannung (doppelte Spitzenspannung), die Scheitelspannung (einfache Spitzenspannung), der Mittelwert des Betrags der Spannung, der Gleichrichtwert, der Effektivwert oder dergleichen sein. Entsprechendes gilt für den fließenden Strom. Ein Kennwert des Stroms kann der Spitzen-Spitzen-Strom (doppelter Spitzenstrom), der Scheitelstrom (einfacher Spitzenstrom), der Mittelwert des Betrags des Stroms, der Gleichrichtwert, der Effektivwert oder dergleichen sein. Ein Kennwert für die Phasenlage kann der Phasenwinkel $\varphi$ zwischen den beiden genannten Kennwerten sein, der die Verschiebung der beiden Kennwerte zueinander beschreibt.

Als Gewebeimpedanz $\underline{Z}$ kann der Quotient aus einem Kennwert der Spannung und einem Kennwert des Stroms mit Bezug auf die Phasenlage genommen werden. Bei einer vorteilhaften Ausführungsform wird dieser Quotient mit einer oder mehreren zuvor ermittelten Quotienten verglichen, um das Erreichen und Durchlaufen eines Gewebewiderstandsminimums zu erkennen. Der Minimumdetektor ist darauf eingerichtet, das Gewebeimpedanzminimum an die Generatorsteuerung zu signalisieren.

$$\underline{Z} = \frac{u(t)}{i(t)} = Z \cdot e^{j\varphi} = Z(cos\varphi + j \cdot sin\varphi) = R + jX;$$

$$|\underline{Z}| = Z, \quad Z = \frac{Up}{Ip} = \frac{Urms}{Irms}$$

In einer anderen Ausführungsform kann der Minimumdetektor darauf eingerichtet sein, den Trend der Gewebeimpedanz anhand gemessener Gewebeimpedanzen zu ermitteln, um eine Prognose für die nächsten zu messenden Gewebeimpedanz zu treffen, wobei das Erreichen und Durchlaufen eines Gewebeimpedanzminimums dann signalisiert wird, wenn die nächste gemessene Gewebeimpedanz Z um einen vorbestimmten Wert über der Prognose für die Nächste zu messende Gewebeimpedanz liegt. Damit kann das Erreichen des Gewebeimpedanzminimums vor dem Wiederanstieg derselben erfasst und die Bildung von Dampfblasen noch schneller unterdrückt werden.

Weitere Ausführungsformen und vorteilhafte Details sind Gegenstand der Zeichnung sowie der Beschreibung und/oder der Patentansprüche. Es zeigen:

Figur 1 die erfindungsgemäße Einrichtung mit einem Generator und einem Instrument in schematischer Darstellung,

Figur 2 die Arbeitsweise des Generators im Diagramm,

Figur 3 ein Zeitdiagramm zur Veranschaulichung der Messung des Gewebewiderstands,

Figur 4 bis 8 weitere Diagramme zur Veranschaulichung der Betriebsweise der Einrichtung in verschiedenen Ausführungsformen,

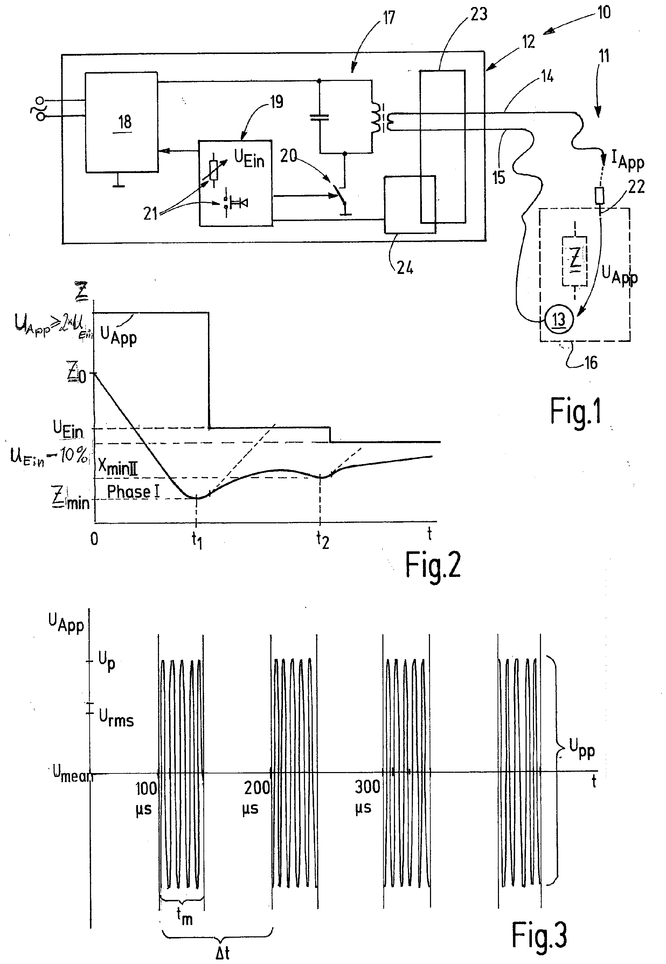

Figur 1 veranschaulicht eine Einrichtung 10 zur Kontaktkoagulation von biologischem Gewebe, zu der ein Instrument 11 und ein die sich speisendes Gerät 12 gehören. Das Instrument 11 und eine zur Stromrückleitung dienende Neutralelektrode 13 sind jeweils über Kabel 14, 15 an das Gerät 12 angeschlossen. Biologisches Gewebe 16, das mittels des Instruments 11 abschnittsweise koaguliert werden soll und das den Stromkreis zwischen dem Instrument 11 und der Neutralelektrode 13 schließt, ist in Figur 1 symbolisch durch einen gestrichelten Block angedeutet. Das biologische Gewebe 16 weist eine Gewebeimpedanz Z auf, die einen ohmschen Anteil R aufweist und mehr oder weniger große reaktive Anteile jX, insbesondere einen kapazitiven Anteil aufweisen kann.

$$R = Z * cos\varphi, \quad X = Z * sin\varphi$$

Das Gerät 12 enthält einen Generator 17 zur Erzeugung hochfrequenter elektrischer Spannungen und Ströme. Der Generator 17 ist an eine Betriebsspannung angeschlossen, die von einem Netzteil 18 bereitgestellt wird. Der Betrieb des Generators 17 wird von einer Generatorsteuerung 19 bestimmt, die zum Beispiel ein elektronisches Schalt- oder Verstärkungselement 20 steuert, um einen zu dem Generator 17 gehörigen Schwingkreis anzuregen. Die Generatorsteuerung 19 kann ein oder mehrere Bedienelemente 21 aufweisen, mittels derer Vorgaben für den Betrieb des Generators gemacht und Einstellungen durch einen Anwender vorgenommen werden können. Beispielsweise kann zu den voreinzustellenden Größen ein Einstellwert $U_{Ein}$ gehören, mit dem der Anwender ihm geläufige Spannungswerte für die Kontaktkoagulation einstellt (zum Beispiel 200 V). Weiter können zu den Einstellwerten die Betriebsart oder sonstige Parameter gehören, beispielsweise, das gewünschte Koagulationsvolumen oder die gewünschte Koagulationszeit, die maximal zu applizierende Energie oder dergleichen. Weiter können sonstige Parameter wie Crestfaktor, Maximalstrom, maximale Leistung und dergleichen einstellbar sein. Der Generator 17 stellt eine Spannung $U_{App}$ bereit, die an der Elektrode 22 des Instruments 11 bereitsteht und auf deren Grund sich ein Strom $I_{App}$ durch das Gewebe 16 ergibt.

Die an das Gewebe angelegte Spannung $U_{App}$ und der durch das Gewebe fließende Strom $I_{App}$ werden von einer Messeinrichtung 23 erfasst und daraus Messwerte abgeleitet. Die Messeinrichtung 23 bestimmt aus dem Messwert des Stroms $I_{App}$ und dem Messwert der Spannung $U_{App}$ die aktuelle Gewebeimpedanz Z. Ein Minimumdetektor 24 ist dabei dazu vorgesehen, zu erkennen, ob oder dass die Gewebeimpedanz Z ein Impedanzminimum erreicht oder durchlaufen hat. In einem solchen Fall signalisiert der Minimumdetektor 24 dies der Generatorsteuerung 19.

Die Generatorsteuerung 19, die Messeinrichtung 23 und der Minimumdetektor 24 sind Funktionsblöcke. Sie können baulich zu einer einzigen Baugruppe integriert oder auf mehrere einzelne Baugruppen aufgeteilt sein. Insbesondere können die Messeinrichtung 23 und der Minimumdetektor 24 mit der Generatorsteuerung zu einer Baugruppe zusammengefasst sein. Die Baugruppen können physische Module oder auch Programmmodule oder dergleichen sein. Beispielsweise kann die Messeinrichtung 23. sowohl den zu messenden Strom $I_{App}$ als auch die zu messende Spannung $U_{App}$ mittels Analog/Digital-Wandler in Datenpaare umwandeln und dann mittels eines Rechenblocks die zugehörigen Gewebeimpedanzwerte bestimmen. Die Gewebeimpedanzwerte können zum Beispiel in einem Speicher zur weiteren Verarbeitung bereitgehalten werden.

Der Minimumdetektor 24 kann dann durch eine Programmroutine gebildet sein, die in den Datenpaaren nach einem Impedanzminimum sucht. Die Gewebeimpedanz kann dabei als Quotient eines der Kennwerte der jeweils gemessenen Spannung $U_{App}$ und eines der Kennwerte des gemessen Stroms $I_{App}$ definiert sein. Figur 3 veranschaulicht die Messung der Spannung $U_{App}$. Beispielsweise wird in geringen Zeitabständen $\Delta t$ von vorzugsweise weniger als 0,2 ms, vorzugsweise in Zeitabständen $\Delta t$ von lediglich 100 μsek die Spannung $U_{App}$ und der Strom $I_{App}$ (d.h. jeweils mindestens ein Kennwert) gemessen. In Figur 3 ist dafür jeweils ein Zeitfenster $t_m$ vorgesehen, das etwas kürzer ist als der Zeitabstand $\Delta t$. Während des Zeitfensters tm wird zumindest ein geeigneter Spannungswert, beispielsweise der Spitzenwert $U_p$, der doppelte Spitzenwert $U_{pp}$, der Mittelwert des Betrags der Spannung Umean, der Effektivwert Urms oder ein ähnlicher Kennwert für die Spannung gemessen. Entsprechend wird ein Kennwert für den Strom gemessen. Dies kann wiederum der Stromspitzenwert $I_p$, der Strombetragsmittelwert $I_{mean}$ oder der Effektivstrom $I_{rms}$ sein. Ferner wird ein Kennwert für die Phasenlage gemessen. Dies kann der Verschiebungswinkel $\varphi$ zwischen der Spannung und dem Strom sein. Für jede Messintervalle gibt sich somit die Gewebeimpedanz als Quotient eines der gemessenen Kennwerte für die Spannung $U_{App}$ (zum Beispiel $U_p$, $U_{pp}$, $U_{mean}$ oder $I_{rms}$) und ein Kennwert des Stroms $I_{App}$ (zum Beispiel $I_p$, $I_{pp}$, $I_{mean}$ oder $I_{rms}$) mit Bezug auf die Phasenlage der gemessenen Kennwerte für die Spannung und den Strom.

Die Generatorsteuerung 19 ist darauf eingerichtet, anhand der Mess- bzw. Rechenwerte für die Gewebeimpedanz Z verschiedene Generatorspannungen $U_{App}$ festzulegen, wie es prinzipiell in Figur 2 veranschaulicht ist. Zu Beginn der Kontaktkoagulation hat das noch nicht beeinflusste biologische Gewebe eine Anfangsimpedanz Zo. Der Generator 17 arbeitet mit einer Spannung $U_{App}$, die von der Generatorsteuerung entsprechend dem Einstellwert $U_{Ein}$ vorgegeben ist. Ist der Einstellwert $U_{Ein}$ beispielsweise auf den für die Kontaktkoagulation üblichen Funkenbildung normalerweise unterbindenden Wert von 200 V festgelegt, gibt die Generatorsteuerung 19 nun einen deutlich höheren, vorzugsweise mindestens doppelt so großen Wert von zum Beispiel 400 V oder darüber als Applikationsspannung $U_{App}$ vor. Entsprechend liefert der Generator 17 eine Spannung $U_{App}$ von 400 V oder darüber. Der sich einstellende hohe Strom $I_{App}$ führt zu einer schnellen Erwärmung des Gewebes 16, womit ein steiler Abfall der Gewebeimpedanz Z auftritt. Erfasst der Minimumdetektor 24 zu einem Zeitpunkt t1 oder kurz danach das Durchlaufen des Impedanzminimums $Z_{min}$, gibt er ein entsprechendes Signal an die Generatorsteuerung 19, damit diese die an das Gewebe 16 angelegte Spannung $U_{App}$ reduziert. Bei der Erfindung wird die Spannung $U_{App}$ auf den Einstellwert $U_{Ein}$ reduziert. Damit wird Funkenbildung unterbunden, wie sie nach Durchlaufen des Impedanzminimums $\underline{Z}_{min}$ aufgrund beginnender Siedevorgänge und entsprechender elektrischer Durchschläge von Dampfblasen möglich wäre. Somit wird auch ein steilerer Anstieg der Gewebeimpedanz unterbunden, wie er durch vorzeitiges Austrocknen von Gewebe entstehen könnte. Bildet sich beispielsweise zu dem Zeitpunkt t2 ein weiteres Impedanzminimum $\underline{Z}_{minII}$ heraus, wird dieses wieder von dem Minimumdetektor 24 erfasst und an die Generatorsteuerung 19 gemeldet, so dass diese die applizierte Spannung $U_{App}$ erneut und somit weiter reduziert, beispielsweise um 10%.

Der Vorgang kann fortgesetzt werden bis die applizierte Spannung $U_{App}$ einen unteren Grenzwert erreicht, der zum Beispiel 60 % des Einstellwerts $U_{Ein}$ betragen kann. Ist dies der Fall kann die Generatorsteuerung die Aktivierung durch Steuerung des elektronischen Schaltelements 20 abbrechen. Die Koagulation ist damit beendet.

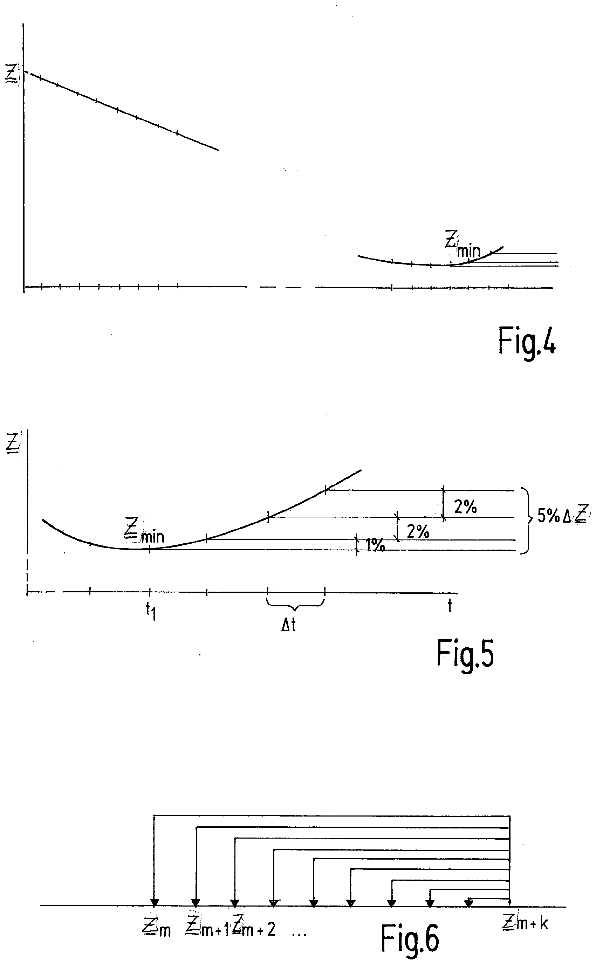

Der Minimumdetektor 24 kann das Impedanzminimum nach jedem geeigneten Verfahren zur Auswertung von Messreihen bestimmen. Figur 4 und 5 veranschaulichen dabei die Verhältnisse in der Nähe des Impedanzminimums. Es wird dabei zur Veranschaulichung zunächst von einem idealen Signalverlauf ausgegangen: Nach Erreichen eines Impedanzminimums $Z_{min}$ zu einem Zeitpunkt t1 steigt die Gewebeimpedanz Z wieder an, wobei die prozentualen Zunahmen von Schritt zu Schritt relativ gering sein können. Außerdem kann den Messwerten eine Messunsicherheit, d.h. ein Rauschen überlagert sein, so dass die geringen einzelnen von Schritt zu Schritt auftretenden Impedanzzunahmen zur Minimumbestimmung untauglich sind. Dies gilt umso mehr, je geringer die Zeitabstände $\Delta t$ zwischen aufeinanderfolgenden Abtastvorgängen sind. Um das Signalrauschen unwirksam zu machen, wird zum Beispiel eine solche Impedanzzunahme als Kriterium für den Wiederanstieg der Impedanz festgelegt, die im normalen Signalrauschen nicht vorkommt, das Signalrauschen also übersteigt. Eine solche Impedanzzunahme kann beispielsweise eine Schwelle von

$$\frac{\Delta Z}{\Delta t} = \frac{Z_{m+1} - Z_m}{t_{m+1} - t_m} \geq 5\%$$

5% sein, die somit in aufeinander folgenden Schritten nicht vorkommt.

Zur Erkennung der Zunahme der Gewebeimpedanz kann die aktuell gemessene Gewebeimpedanz $\underline{Z}_{m+k}$ gemäß Figur 6 mit einer Reihe davorliegender Impedanzmesswerte Zm, Zm+i, $\underline{Z}_{m+2}$ usw. verglichen werden. Auf einen Wiederanstieg der Gewebeimpedanz, d.h. das Durchlaufen eines Minimums, kann der Minimumdetektor 24 schließen, wenn

der Impedanzwert $\underline{Z}_{m+k}$ um wenigstens 5% oder eine sonstige vorgegebene Impedanzzunahmeschwelle $\Delta Z$ größer ist als zumindest einer der vorausliegenden Gewebeimpedanzwerte. Es kann auch festgelegt werden, dass der Minimumdetektor 24 ein Minimum $Z_{min}$ nur dann erfasst, wenn der aktuelle Werte $\underline{Z}_{m+k}$ größer ist als zumindest zwei oder mehrere vorausliegender Impedanzwerte.

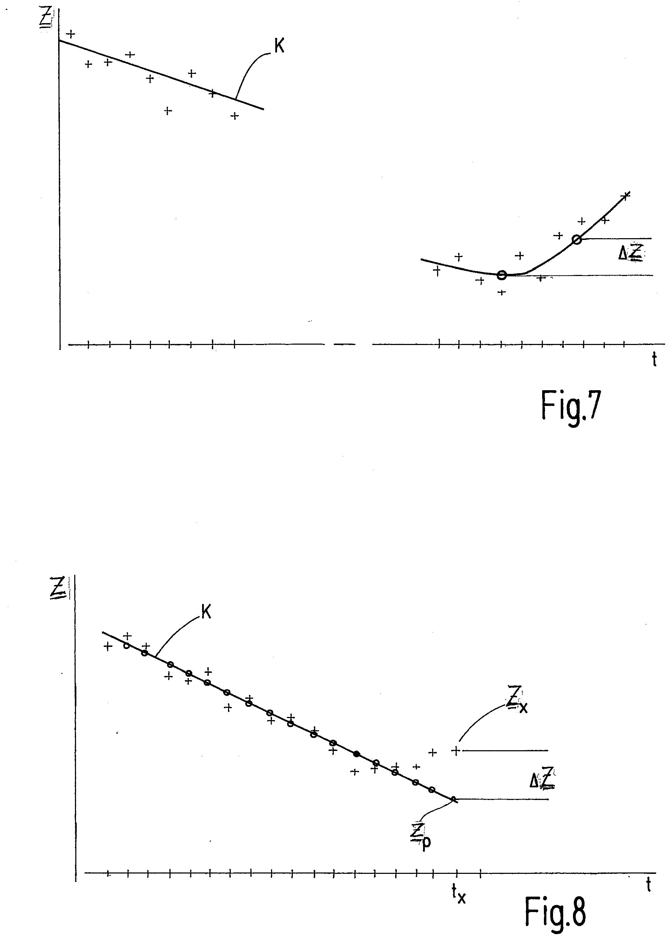

Eine Abwandlung der Minimumserfassung durch den Minimumdetektor 24 ist in Figur 7 veranschaulicht. Es sind dort als Kreuze einzelne Impedanzwerte dargestellt, die durch Strom- und Spannungsmessung und darauf aufbauend nachfolgende Impedanzberechnung ermittelt worden sind. Diese Impedanzwerte unterliegen zufälligen Schwankungen, die sich aus der im mikroskopischen Maßstab vorhandenen Inhomogenität des biologischen Gewebes und den dort ablaufenden Denaturierungsvorgängen ergeben können. Der Impedanzdetektor 24 und/oder die Messeinrichtung 23 können so ausgelegt sein, dass sie aufgrund der Einzelmesswerte eine Messkurve K bestimmen, die den Verlauf der Impedanz Z über der Zeit t annähert. Die Messkurve K kann durch Splines n-ten Grades oder nach Best-Fit-Algorithmen, zum Beispiel der Methode des kleinsten Fehlerquadrats oder anderen geeigneten Mitteln, bestimmt werden. Als Messkurve K kommen Polynome, Geraden, Parabeln sowie andere gekrümmte Kurven und Zusammensetzungen aus diesen in Betracht. Beispielsweise können im Wesentlichen lineare Messwertverläufe durch Geraden und nicht lineare Messwertverläufe durch Parabelabschnitte angenähert werden. Die Überschreitung eines maximalen Impedanzanstiegs $\Delta Z$ kann dann anhand des Verlaufs der Messkurve K ermittelt werden. Dies kann erfolgen, indem erfasst wird, dass der Anstieg der Kurve K positiv ist. Zur Erfassung dieses Umstandes kann erfasst werden, ob und dass ein positiver Impedanzanstieg $\Delta Z$ vorhanden ist, der einen Grenzwert von z.B. 5% der niedrigsten Gewebeimpedanz $\underline{Z}_{min}$ übersteigt.

Die vorgenannten Verfahren erfassen das Impedanzminimum nach dessen Durchlaufen, durch den sich einstellenden Wiederanstieg der Impedanz. Es ist jedoch auch möglich, das Erreichen des Minimums bzw. das Durchlaufen desselben zu einem früheren Zeitpunkt festzustellen. Dies ist in Figur 8 veranschaulicht. Die im Diagramm vorhandenen kleinen Kreuze symbolisieren die zu jedem Zeitpunkt t jeweils bestimmten Gewebeimpedanzen. Die Messkurve K ist eine Regressionsgerade. Auf ihr liegen idealisierte Impedanzwerte, die jeweils durch einen kleinen Kreis angedeutet sind. Der Minimumdetektor bestimmt diese idealisierten, d.h. bei idealem Gewebeverhalten zu erwartenden Impedanzwerte und vergleicht jeweils den letzten idealisierten Impedanzwert mit dem letzten gemessenen Impedanzwert. In Figur 8 ist zu einem Zeitpunkt $t_x$ die Gewebeimpedanz $\underline{Z}_x$ bestimmt worden. Aufgrund der Kurve K ergibt sich die Impedanzprognose $Z_p$. Die Differenz $\Delta Z$ zwischen der Impedanzprognose $Z_p$ und der tatsächlichen Gewebeimpedanz $\underline{Z}_x$ erreicht oder überschreitet eine Schwelle von z.B. wieder 5% des Prognosewertes $Z_p$. Der Minimumdetektor kann darauf ausgelegt sein, in Reaktion darauf das Erreichen und Durchlaufen des Minimums $\underline{Z}_{min}$ der Gewebeimpedanz Z anzuzeigen. Anstelle der oben genannten 5%-Schwelle können andere Schwellwerte und Kriterien festgelegt sein.

Der vorgestellte Generator 12 stellt einen Wiederanstieg der Gewebeimpedanz Z fest, der auf ein Beginnen des Austrocken des Gewebes 16 und somit auch Dampfbildung hindeutet. Durch Reduktion der angelegten Spannung $U_{App}$ wird Funkenbildung vermieden. Andererseits wird zu Beginn der Bestromung des biologischen Gewebes 16 mit überhöhter Spannung (zum Beispiel $U_{App} \geq 2{*}U_{Ein}$) gearbeitet, wodurch eine sehr schnelle Koagulation erfolgt. Die sonst bei überhöhter Spannung auftretenden nachteiligen Erscheinungen wie Zerreißen von Gewebe, Aufplatzen von Gefäßen, unerwünschte Funkenbildung und somit Karbonisierung werden vermieden.

Eine erfindungsgemäße Einrichtung 10 umfasst ein Gerät 12 zur Bereitstellung von hochfrequenter Spannung $U_{App}$ zur Kontaktkoagulation von biologischem Gewebe 16. Das Gerät 12 ist darauf eingerichtet, zu Beginn der Kontaktkoagulation mit einer hohen normalerweise zur Kontaktkoagulation nicht geeigneten, sondern sonst zur Funkenkoagulation verwendeten Spannung von zum Beispiel mehr als 400 V zu arbeiten. Während des Betriebs des Geräts 12 wird die Gewebeimpedanz Z überwacht. Dies kann durch fortwährendes Messen der Spannung $U_{App}$ und des fließenden Stroms $I_{App}$ geschehen. Aus beiden bestimmt die Messeinrichtung 23 fortwährend die Gewebeimpedanz Z. Ein Minimumdetektor 24 ist dazu vorgesehen, das Durchlaufen eines Impedanzminimums Zmin festzustellen und, falls solches festgestellt wird, die Generatorsteuerung 19 zu veranlassen, die von dem Gerät 12 abgegebene Spannung $U_{App}$ auf einen Wert zu reduzieren, der Funkenbildung sowie Austrocknen des Gewebes vermeidet.

Bezugszeichen:

| | |
|---|---|
| 10 | Einrichtung |
| 11 | Instrument |
| 12 | Gerät |
| 13 | Neutralelektrode |
| 14, 15 | Kabel |
| 16 | biologisches Gewebe |
| $\underline{Z}$ | Gewebeimpedanz |

(fortgesetzt)

| | |
|---|---|
| 17 | Generator |
| 18 | Netzteil |
| 19 | Generatorsteuerung |
| 20 | Verstärker- oder Schaltelement |
| 21 | Bedienelemente |
| $U_{Ein}$ | Einstellwert für die HF-Spannung |
| $U_{App}$ | Applizierte HF-Spannung zu einem bestimmten Zeitpunkt |
| $I_{App}$ | Strom durch das Gewebe 16 |
| 22 | Elektrode des Instruments 11 |
| 23 | Messeinrichtung |
| 24 | Minimumdetektor |
| $\Delta t$ | Zeitabstand |
| $t_m$ | Zeitfenster |
| $U_p$ | Scheitelwert der Spannung $U_{App}$ |
| $u(t)$ | Zeitlicher Verlauf der Spannung $U_{App}$ |
| $U_{pp}$ | doppelter Spitzenwert der Spannung $U_{App}$ |
| $U_{mean}$ | Mittelwert des Betrags der Spannung $U_{App}$ |
| $U_{rms}$ | Effektivwert der Spannung $U_{App}$ |
| $I_p$ | Scheitelwert des Stroms $I_{App}$ |
| $i(t)$ | Zeitlicher Verlauf des Stroms $I_{App}$ |
| $I_{pp}$ | doppelter Spitzenwert des Stroms $I_{App}$ |
| $I_{mean}$ | Mittelwert des Betrags des Stroms $I_{App}$ |
| $I_{rms}$ | Effektivwert des Stroms $I_{App}$ |
| $\underline{Z}_0$ | Anfangsimpedanz |
| $\underline{Z}_{min}$ | Erstes auftretendes Gewebeimpedanzminimum |
| $\underline{Z}_{minII}$ | Zweites oder weiteres auftretendes Gewebeimpedanzminimum |
| K | Messkurve |
| $\Delta\underline{Z}$ | Impedanzzunahme |
| $\underline{Z}_p$ | Impedanzprognose |
| R | Ohmscher Widerstand |
| X | Blindwiderstand |
| $\varphi$ | Phasenwinkel zwischen Spannung $U_{App}$ und Strom $I_{APP}$ |
| | |

**Patentansprüche**

1. Einrichtung (10) zur Kontaktkoagulation von biologischem Gewebe (16), mit:

einem Generator (17) zur Bereitstellung von HF-Spannung ($U_{App}$) und Abgabe von HF-Strom ($I_{App}$),
einer Generatorsteuerung (19), mittels derer die von dem Generator (17) bereitgestellte HF-Spannung ($U_{App}$) beeinflussbar ist,

einem Instrument (11), mit mindestens einer Elektrode (22), die von dem Generator (17) mit HF-Strom gespeist ist,

einer Messeinrichtung (23) zur Überwachung der Gewebeimpedanz ($\underline{Z}$),

einem Minimumdetektor (24) zur Erkennung eines Minimums ($\underline{Z}_{min}$) der Gewebeimpedanz ($\underline{Z}$),

wobei der Minimumdetektor (24) mit der Generatorsteuerung (19) verbunden ist, um den Generator (17) bei Erkennung eines Minimums ($\underline{Z}_{min}$) der Gewebeimpedanz ($\underline{Z}$) zu veranlassen, danach zur Vermeidung von Dampfbildung eine reduzierte HF-Spannung ($U_{App}$) bereitzustellen,

wobei in der Generatorsteuerung (19) ein Einstellwert ($U_{Ein}$) für die zur funkenfreien Kontaktkoagulation geeignete, zu applizierende Spannung ($U_{App}$) hinterlegt und die Generatorsteuerung (19) darauf eingerichtet ist, zu Beginn eines Koagulationsvorgangs, bei dem das Gewebe seine Anfangsimpedanz ($Z_0$) aufweist, den Generator (17) zu veranlassen, die zu applizierenden Spannung ($U_{App}$) mit einem Wert bereitzustellen, der größer als der Einstellwert ($U_{Ein}$) ist,

wobei die zu Beginn eines Koagulationsvorgangs zu applizierende Spannung ($U_{App}$) mindestens doppelt so groß ist wie der Einstellwert ($U_{Ein}$) und normalerweise nicht zur funkenfreien Kontaktkoagulation geeignet ist, und wobei die erste Spannungsreduktion die Spannung auf den Einstellwert ($U_{Ein}$) reduziert.

2. Einrichtung nach Anspruch 1, **dadurch gekennzeichnet, dass** die Generatorsteuerung (19) darauf eingerichtet ist, in Reaktion auf die Erkennung eines zweiten oder weiteren Minimums ($\underline{Z}_{minII}$) der Gewebeimpedanz (Z) den Generator (17) zu veranlassen, die zu applizierenden Spannung ($U_{App}$) mit einem Wert bereitzustellen, der kleiner als der Einstellwert ($U_{Ein}$) ist.

3. Einrichtung nach Anspruch 2, **dadurch gekennzeichnet, dass** die Generatorsteuerung (19) darauf eingerichtet ist, in Reaktion auf die Erkennung eines zweiten oder weiteren Minimums ($\underline{Z}_{minII}$) der Gewebeimpedanz ($\underline{Z}$) den Generator (17) zu veranlassen, die zu applizierenden Spannung ($U_{App}$) mit einem Wert bereitzustellen, der 10% kleiner als die zuvor gelieferte Spannung ($U_{App}$) ist.

4. Einrichtung nach Anspruch 2 oder 3, **dadurch gekennzeichnet, dass** die Generatorsteuerung (19) darauf eingerichtet ist, die HF-Applikation des Generators zu beenden, wenn die zu applizierenden Spannung ($U_{App}$) einen Wert erreicht hat, der einen festgelegten Bruchteil des eingestellten Wertes ($U_{Ein}$) nicht überschreitet.

5. Einrichtung nach einem der vorstehenden Ansprüche, **dadurch gekennzeichnet, dass** der Einstellwert ($U_{Ein}$) mittels eines Einstellmittels variabel vorgebbar ist.

6. Einrichtung nach einem der vorstehenden Ansprüche, **dadurch gekennzeichnet, dass** die aktuelle Gewebeimpedanz ($\underline{Z}$) in Zeitabständen ($\Delta t$) erfasst wird, die geringer als 0,2 ms, vorzugsweise höchstens 0,1 ms sind.

7. Einrichtung nach Anspruch 6, **dadurch gekennzeichnet, dass** die Messeinrichtung (23) darauf eingerichtet ist, die Erfassung der Gewebeimpedanz ($\underline{Z}$) in Abtastfenstern ($t_m$) vorzunehmen, deren Länge mindestens so groß ist, wie eine Schwingungsperiode der Spannung ($U_{App}$).

8. Einrichtung nach Anspruch 7, **dadurch gekennzeichnet, dass** die Messeinrichtung (23) darauf eingerichtet ist, während der Messperiode mindesten einen Kennwert der anliegenden Spannung ($U_{App}$) , mindestens einen Kennwert des fließenden Stroms ($I_{App}$)und mindestens einen Kennwert der Phasenlage ($\varphi$) zwischen anliegender Spannung und fließenden Strom zu erfassen und daraus einen Quotienten mit Bezug auf die Verschiebung der Phasenlage zwischen anliegender Spannung und fließendem Strom zu bilden, der die Gewebeimpedanz ($\underline{Z}$) kennzeichnet.

9. Einrichtung nach einem der Ansprüche 6 bis 8, **dadurch gekennzeichnet, dass** die Messeinrichtung (23) darauf eingerichtet ist, aus mehreren in Zeitabständen ($\Delta t$) erfassten Gewebewiderständen ($\underline{Z}$) einen laufenden Mittelwert zu bilden.

10. Einrichtung nach Anspruch 8, **dadurch gekennzeichnet, dass** der Minimumdetektor (24) darauf eingerichtet ist, bei Feststellung eines Anstiegs der Gewebeimpedanz ($\underline{Z}$) gegenüber einem zuvor gemessenen Gewebeimpedanz ($\underline{Z}$) das Erreichen und Durchlaufen eines Gewebeimpedanzminimums ($\underline{Z}_{min}$) zu signalisieren.

11. Einrichtung nach Anspruch 8, **dadurch gekennzeichnet, dass** der Minimumdetektor (24) darauf eingerichtet ist, bei Feststellung eines Anstiegs der Gewebeimpedanz ($\underline{Z}$) oder eines geglätteten Mittelwerts der Gewebeimpedanz gegenüber der geringsten Gewebeimpedanz einer Gruppe zuvor gemessener Gewebewiderstände das Erreichen und Durchlaufen eines Gewebeimpedanzsminimums ($\underline{Z}_{min}$) zu signalisieren.

**12.** Einrichtung nach einem der vorstehenden Ansprüche, **dadurch gekennzeichnet, dass** der Minimumdetektor (24) darauf eingerichtet ist, den Trend des Verlaufs der Gewebeimpedanz ($\underline{Z}$) zu ermitteln, um eine Prognose ($\underline{Z}_p$) für die nächsten zu messende Gewebeimpedanz zu treffen, wobei das Erreichen und Durchlaufen eines Gewebeimpedanzsminimums ($\underline{Z}_{min}$) signalisiert wird, wenn die nächste gemessene Gewebeimpedanz ($\underline{Z}$) um einen vorbestimmten Wert ($\Delta\underline{Z}$) über dem nächsten zu messenden Gewebeimpedanz ($\underline{Z}$) liegt.

**Claims**

**1.** Arrangement (10) for contact coagulation of biological tissue (16), with:

- a generator (17) for provision of HF voltage ($U_{App}$) and the emission of HF current ($I_{App}$),
- a generator controller (19), by means of which the HF voltage ($U_{App}$) provided by the generator can be influenced,
- an instrument (11) with at least one electrode (22) which is supplied with HF current by the generator (17),
- a measuring device (23) for monitoring the tissue impedance ($\underline{Z}$),
- a minimum detector (24) for detecting a minimum ($\underline{Z}_{min}$) of the tissue impedance ($\underline{Z}$),
wherein the minimum detector (24) is connected to the generator controller (19) in order to induce the generator (17), on detection of a minimum ($\underline{Z}_{min}$) of the tissue impedance ($\underline{Z}$), then to provide a reduced HF voltage ($U_{App}$) so as to avoid vapour formation,
wherein a setting value ($U_{Ein}$) for the voltage ($U_{App}$) to be applied, which is suitable for spark-free contact coagulation, is stored in the generator controller (19) and the generator controller (19) is configured, at the start of a coagulation process during which the tissue has its initial impedance ($\underline{Z}_o$), to induce the generator (17) to provide the voltage ($U_{App}$) to be applied with a value which is greater than the setting value ($U_{Ein}$),
wherein the voltage ($U_{App}$) to be applied at the start of a coagulation process is at least twice as great as the setting value ($U_{Ein}$) and not normally suitable for spark-free contact coagulation,
and wherein the first voltage reduction reduces the voltage to the setting value ($U_{Ein}$).

**2.** Arrangement according to claim 1, **characterised in that** the generator controller (19) is configured, in response to the detection of a second or further minimum ($\underline{Z}_{minII}$) of the tissue impedance ($\underline{Z}$), to induce the generator (17) to provide the voltage ($U_{App}$) to be applied with a value which is lower than the setting value ($U_{Ein}$).

**3.** Arrangement according to claim 2, **characterised in that** the generator controller (19) is configured, in response to the detection of a second or further minimum ($\underline{Z}_{minII}$) of the tissue impedance ($\underline{Z}$), to induce the generator (17) to provide the voltage ($U_{App}$) to be applied with a value which is 10% lower than the previously supplied voltage ($U_{App}$).

**4.** Arrangement according to claim 2 or 3, **characterised in that** the generator voltage (19) is configured to end the HF application of the generator when the voltage ($U_{App}$) to be applied has reached a value which does not exceed an established fraction of the set value ($U_{Ein}$).

**5.** Arrangement according to any of the preceding claims, **characterised in that** the setting value ($U_{Ein}$) is variably predefinable by means of a setting means.

**6.** Arrangement according to any of the preceding claims, **characterised in that** the actual tissue impedance ($\underline{Z}$) is detected at time intervals ($\Delta t$) which are shorter than 0.2 ms, preferably at most 0.1 ms.

**7.** Arrangement according to claim 6, **characterised in that** the measuring device (23) is configured to carry out the detection of tissue impedance ($\underline{Z}$) in sampling windows ($t_m$), the length of which is at least as great as the oscillation period of the voltage ($U_{App}$).

**8.** Arrangement according to claim 7, **characterised in that** the measuring device (23) is configured, during the measuring period, to detect at least one characteristic value of the applied voltage ($U_{App}$), at least one characteristic value of the flowing current ($I_{App}$), and at least one characteristic value of the phase position ($\varphi$) between applied voltage and flowing current, and from these to form a quotient relating to the shift in phase position between applied voltage and flowing current which characterises the tissue impedance ($\underline{Z}$).

**9.** Arrangement according to any of claims 6 to 8, **characterised in that** the measuring device (23) is configured to form an ongoing mean value from several tissue resistances ($\underline{Z}$) detected at time intervals ($\Delta t$).

**10.** Arrangement according to claim 8, **characterised in that** the minimum detector (24) is configured to signal the reaching and passing of a tissue impedance minimum ($\underline{Z}_{min}$) on establishing a rise in tissue impedance ($\underline{Z}$) in relation to a previously measured tissue impedance ($\underline{Z}$).

**11.** Arrangement according to claim 8, **characterised in that** the minimum detector (24) is configured to signal the reaching and passing of a tissue impedance minimum ($\underline{Z}_{min}$) on establishing a rise in tissue impedance ($\underline{Z}$) or in a smoothed mean value of the tissue impedance in relation to the lowest tissue impedance of a group of tissue resistances previously measured.

**12.** Arrangement according to any of the preceding claims, **characterised in that** the minimum detector (24) is configured to determine the trend of the development of tissue impedance ($\underline{Z}$) in order to make a prognosis ($\underline{Z}_p$) for the next tissue impedance to be measured, wherein the reaching and passing of a tissue impedance minimum ($\underline{Z}_{min}$) is signalled when the next measured tissue impedance ($\underline{Z}$) lies by a predefined value ($\Delta\underline{Z}$) above the next tissue impedance ($\underline{Z}$) to be measured.

**Revendications**

**1.** Dispositif (10) destiné à la coagulation par contact de tissu biologique (16), comprenant

un générateur (17) pour la fourniture d'une tension HF ($U_{App}$) et la délivrance de courant HF ($I_{App}$),
une commande de générateur (19) qui permet d'influencer la tension HF ($U_{App}$) fournie par le générateur (17),
un instrument (11) comportant au moins une électrode (22) qui est alimentée en courant HF par le générateur (17),
un dispositif de mesure (23) destiné à surveiller l'impédance de tissu ($\underline{Z}$),
un détecteur de minimum (24) destiné à détecter un minimum ($\underline{Z}_{min}$) de l'impédance de tissu ($\underline{Z}$),
le détecteur de minimum (24) étant relié à la commande de générateur (19) pour faire en sorte qu'en cas de détection d'un minimum ($\underline{Z}_{min}$) de l'impédance de tissu ($\underline{Z}$), le générateur (17) fournisse ensuite une tension HF ($U_{App}$) réduite, afin d'éviter la formation de vapeur,
sachant qu'une valeur de réglage ($U_{Ein}$) pour la tension ($U_{App}$) appropriée, devant être appliquée en vue de la coagulation par contact sans étincelles, est déposée dans la commande de générateur (19), et que la commande de générateur (19) est conçue pour faire en sorte qu'au début d'un processus de coagulation lors duquel le tissu présente son impédance initiale ($Z_0$), le générateur (17) fournisse la tension ($U_{App}$) à appliquer, avec une valeur qui est supérieure à la valeur de réglage ($U_{Ein}$),
sachant que la tension ($U_{App}$) devant être appliquée au début d'un processus de coagulation correspond au moins au double de la valeur de réglage ($U_{Ein}$) et ne convient normalement pas pour une coagulation par contact sans étincelles,
et sachant que la première réduction de tension abaisse la tension jusqu'à la valeur de réglage ($U_{Ein}$).

**2.** Dispositif selon la revendication 1, **caractérisé en ce que** la commande de générateur (19) est conçue pour faire en sorte qu'en réaction à la détection d'un deuxième ou d'un autre minimum ($\underline{Z}_{minII}$) de l'impédance de tissu ($\underline{Z}$), le générateur (17) fournisse la tension ($U_{App}$) à appliquer, avec une valeur qui est inférieure à la valeur de réglage ($U_{Ein}$).

**3.** Dispositif selon la revendication 2, **caractérisé en ce que** la commande de générateur (19) est conçue pour faire en sorte qu'en réaction à la détection d'un deuxième ou d'un autre minimum ($\underline{Z}_{minII}$) de l'impédance de tissu ($\underline{Z}$), le générateur (17) fournisse la tension ($U_{App}$) à appliquer, avec une valeur qui est inférieure de 10 % à la tension ($U_{App}$) fournie précédemment.

**4.** Dispositif selon la revendication 2 ou 3, **caractérisé en ce que** la commande de générateur (19) est conçue pour mettre fin à l'application HF du générateur lorsque la tension ($U_{App}$) à appliquer a atteint une valeur qui ne dépasse pas une fraction définie de la valeur réglée ($U_{Ein}$).

**5.** Dispositif selon l'une des revendications précédentes, **caractérisé en ce que** la valeur de réglage ($U_{Ein}$) peut être prédéterminée de façon variable à l'aide d'un moyen de réglage.

**6.** Dispositif selon l'une des revendications précédentes, **caractérisé en ce que** l'impédance de tissu ($\underline{Z}$) actuelle est mesurée à des intervalles de temps ($\Delta t$) qui sont inférieurs à 0,2 ms et sont de préférence au maximum de 0,1 ms.

**7.** Dispositif selon la revendication 6, **caractérisé en ce que** le dispositif de mesure (23) est conçu pour réaliser la mesure de l'impédance de tissu ($\underline{Z}$) à l'intérieur de fenêtres d'interrogation ($t_m$) dont la longueur est au moins égale à une période de la tension ($U_{App}$).

**8.** Dispositif selon la revendication 7, **caractérisé en ce que** le dispositif de mesure (23) est conçu pour recueillir pendant la période de mesure, au moins une valeur caractéristique de la tension ($U_{App}$) appliquée, au moins une valeur caractéristique du courant ($I_{App}$) qui circule et au moins une valeur caractéristique de la relation de phase ($\varphi$) entre la tension appliquée et le courant qui circule, et d'établir à partir de ces valeurs un quotient en rapport avec le décalage de la relation de phase entre la tension appliquée et le courant qui circule, caractérisant l'impédance du tissu ($\underline{Z}$).

**9.** Dispositif selon l'une des revendications 6 à 8, **caractérisé en ce que** le dispositif de mesure (23) est conçu pour former une valeur moyenne continue à partir de plusieurs impédances de tissu ($\underline{Z}$) recueillies à des intervalles de temps ($\Delta t$).

**10.** Dispositif selon la revendication 8, **caractérisé en ce que** le détecteur de minimum (24) est conçu pour signaler l'atteinte et le passage par un minimum d'impédance de tissu ($\underline{Z}_{min}$) lorsqu'une augmentation de l'impédance de tissu ($\underline{Z}$) est constatée par rapport à une impédance de tissu ($\underline{Z}$) mesurée précédemment.

**11.** Dispositif selon la revendication 8, **caractérisé en ce que** le détecteur de minimum (24) est conçu pour signaler l'atteinte et le passage par un minimum d'impédance de tissu ($\underline{Z}_{min}$) lorsqu'une augmentation de l'impédance de tissu ($\underline{Z}$) ou d'une valeur moyenne lissée de l'impédance de tissu est constatée par rapport à l'impédance de tissu la plus faible d'un groupe de résistances de tissu mesurées précédemment.

**12.** Dispositif selon l'une des revendications précédentes, **caractérisé en ce que** le détecteur de minimum (24) est conçu pour déterminer la tendance de l'évolution de l'impédance de tissu ($\underline{Z}$), aux fins d'établir un pronostic ($\underline{Z}_p$) pour l'impédance de tissu suivante à mesurer, en signalant l'atteinte et le passage par un minimum d'impédance de tissu ($\underline{Z}_{min}$), lorsque l'impédance de tissu ($\underline{Z}$) suivante mesurée est supérieure d'une valeur prédéterminée ($\Delta Z$) à l'impédance de tissu ($\underline{Z}$) suivante à mesurer.

Fig.1

Fig.2

Fig.3

Fig.4

Fig.5

Fig.6

Fig.7

Fig.8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2520240 A1
- EP 1862137 A1
- US 2003158551 A1
- RU 2294171 C2
- DE 3622337 A1